# EUROPÄISCHE PATENTSCHRIFT

(11) **EP 1 562 499 B1**
(45) Veröffentlichungstag und Bekanntmachung des Hinweises auf die Patenterteilung: **26.04.2006**
(21) Anmeldenummer: 02807752.7
(22) Anmeldetag: 04.09.2002
(51) Int. Cl.: A61B 17/70

(54) **ORTHOPÄDISCHE FIXATIONSEINRICHTUNG**
ORTHOPEDIC FIXATION DEVICE
DISPOSITIF DE FIXATION ORTHOPEDIQUE

(43) Veröffentlichungstag der Anmeldung: 17.08.2005
(73) Patentinhaber: Aesculap AG & Co. KG, 78532 Tuttlingen (DE)
(72) Erfinder: KRAMER, Ulrich, 78532 Tuttlingen (DE); SCHUMACHER, Jörg, 78532 Tuttlingen (DE); POTULSKI, Michael, 82441 Ohlstadt (DE); BEISSE, Rudolf, 82418 Seehausen (DE); KOZAK, Jeffrey, Houston, TX 77030-4509 (US)
(74) Vertreter: Boehme, Ulrich
(86) Internationale Anmeldenummer: PCT/EP2002/009879
(87) Internationale Veröffentlichungsnummer: WO 2004/021902

(56) Entgegenhaltungen:
- US-A- 5 474 551
- US-A- 5 476 463
- US-A- 5 876 402

## Beschreibung

Die Erfindung betrifft eine orthopädische Fixationseinrichtung mit einem zumindest abschnittsweise schaftförmigen Fixationsglied, einem schaftförmigen Lagerkörper und mit einem das Fixationsglied und den Lagerkörper seitlich versetzt zueinander festlegenden Querträger, in dem das Fixationsglied um seine Längsachse verdrehbar und in einer bestimmten Winkelstellung festklemmbar ist.

Eine Fixationseinrichtung ist beispielsweise aus dem US-Patent 5,545,228 bekannt, dort trägt ein Fixationsglied in Form einer Knochenschraube einen fest mit ihr verbundenen Querträger, an dessen anderem Ende ein Lagerkörper in Form eines Außengewindezapfens angeordnet ist. Mit dieser Anordnung ist es möglich, den Lagerkörper seitlich versetzt zum Fixationsglied anzuordnen. Dadurch erhält der Operateur beispielsweise bei einer orthopädischen Fixationseinrichtung eine größere Freiheit, den Außengewindezapfen gegenüber der Knochenschraube zu positionieren, es genügt dazu, die Knochenschraube in unterschiedliche Winkelstellung in den Knochen einzuschrauben. Allerdings ist dazu notwendig, zur Verstellung der Position des Querträgers auch die Knochenschraube im Knochen zu verdrehen, und dies kann zu einer unerwünschten Lockerung einer einmal eingedrehten Schraube führen.

In den US-Patenten 5,474,551, 5,476,463 und 5,876,402 sind weitere orthopädische Fixationseinrichtungen beschrieben, bei denen das Fixationsglied in einer bestimmten Winkelstellung festklemmbar ist.

Es ist Aufgabe der Erfindung, eine gattungsgemäße orthopädische Fixationseinrichtung so weiterzubilden, daß einerseits eine Verlagerung der Position des Lagerkörpers relativ zu dem Fixationsglied auch möglich ist, ohne das Fixationsglied in seiner Fixationsstellung verändern zu müssen, und andererseits eine besonders effektive Klemmung ermöglicht wird.

Diese Aufgabe wird bei einer orthopädischen Fixationseinrichtung der eingangs beschriebenen Art erfindungsgemäß dadurch gelöst, daß zur Festklemmung im Querträger ein Klemmstück gegen das Fixationsglied verschiebbar ist.

Insbesondere kann zur Verschiebung des Klemmstückes im Querträger ein am Klemmstück anliegendes, um eine Drehachse verdrehbares Spannglied gelagert sein. Man erhält auf diese Weise eine sehr kompakte Anordnung, mit der der Querträger relativ zum Fixationsglied gelöst oder festgelegt werden kann. Es ist dabei vorteilhaft, wenn der Drehwinkel des Spanngliedes in einer oder in beiden Richtungen begrenzt ist, beispielsweise durch einen Anschlag. Auf diese Weise kann insbesondere vermieden werden, daß das Klemmstück zu kräftig gegen das Fixationsglied gespannt wird, dadurch könnten Beschädigungen der gesamten Einrichtung eintreten. Die definierte Verdrehbarkeit zwischen zwei Endpunkten erleichtert auch für den Operateur die Handhabung der Einrichtung.

Die Drehachse des Spanngliedes liegt vorzugsweise senkrecht auf der Ebene des Querträgers, vorteilhaft ist es dabei, wenn die Drehachse des Spanngliedes parallel zur Längsachse des Fixationsgliedes verläuft.

Eine besonders vorteilhafte Konstruktion ergibt sich, wenn der Querträger ein Langloch aufweist, in welchem das Fixationsglied, das Klemmstück und das Spannglied aufgenommen sind. Dadurch ergibt sich eine besonders platzsparende und kompakte Anordnung, die es ermöglicht, gegebenenfalls das Fixationsglied und den Lagerkörper auch sehr dicht nebeneinander anzuordnen.

Eine besonders platzsparende Anordnung erhält man dann, wenn gemäß einer bevorzugten Ausführungsform die Drehachse des Spanngliedes und die Längsachse des Lagerkörpers zusammenfallen. Das Spannglied ist dann konzentrisch zum Lagerkörper angeordnet, man benötigt für die Anordnung und die Verdrehung des Spanngliedes keinen zusätzlichen Platz mehr.

Beispielsweise kann der Lagerkörper eine fest mit dem Querträger verbundene Hülse sein, durch die hindurch ein Werkzeug zur Verdrehung des Spanngliedes einsetzbar ist.

Dabei ist es vorteilhaft, wenn der Lagerkörper Aufnahmen für ein Gegenhalterwerkzeug aufweist, mit dessen Hilfe der Lagerkörper beim Spannen des Spanngliedes festgehalten werden kann, so daß auf den Querträger beim Spannen des Spanngliedes keine unerwünschten Drehmomente ausgeübt werden.

Bei einer anderen Ausführungsform kann vorgesehen sein, daß das Spannglied ein im Querträger verdrehbarer Ring ist, der am querträgerseitigen Ende des Lagerkörpers angeordnet ist.

Das Spannglied kann bei einer anderen Ausführungsform auch an einem im Lagerkörper drehbar gelagerten Kern angeordnet sein, der sich zumindest über einen Teil seiner Länge innerhalb des Lagerkörpers erstreckt.

Bei einer abgewandelten Ausführungsform ist vorgesehen, daß der Lagerkörper im Querträger um seine Längsachse verdrehbar ist und selbst das Spannglied trägt.

Es ist vorteilhaft, wenn das Spannglied im Querträger in axialer Richtung festgelegt ist.

Bei einer besonders bevorzugten Ausführungsform ist vorgesehen, daß das Spannglied einen zu seiner Drehachse exzentrisch angeordneten Exzenter aufweist, der an dem Klemmstück anliegt.

Bei einer anderen bevorzugten Ausführungsform weist das Spannglied eine geneigte, am Klemmstück anliegende Bahnkurve auf.

Es kann dabei vorgesehen sein, daß die Bahnkurve das Klemmstück bei Verdrehung des axial unverschieblichen Spanngliedes parallel zur Drehachse gegen das Fixationsglied verschiebt und dieses dadurch festklemmt.

Vorteilhaft ist es, wenn das Fixationsglied einen sich erweiternden Schaft aufweist, an dem das Klemmstück anliegt, und axial unverschieblich und frei verdrehbar im Querträger gelagert ist. Durch die axiale Verschiebung des Klemmstückes erfolgt dadurch eine Festlegung des Fixationsgliedes.

Auch eine Überlagerung- der Wirkung eines Exzenters und eines in axialer Richtung wirkenden Spanngliedes ist möglich.

Bei einer abgewandelten Ausführungsform kann auch vorgesehen sein, daß das Fixationsglied durch eine kugelige Verbindung mit dem Querträger verschwenkbar verbunden ist und das Klemmstück beim Fixieren in Richtung auf eine kugelige Lagerfläche des Fixationsgliedes verschiebbar ist.

Das Fixationsglied kann ein beliebiger schaftförmiger, hakenförmiger oder zapfenförmiger Bestandteil einer Fixationseinrichtung sein, besonders bevorzugt wird das Fixierglied als Knochenschraube oder als Haken ausgeführt.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer orthopädischen Fixationseinrichtung zur Festlegung eines stangenförmigen Verbindungsgliedes;
- Figur 2:: eine Längsschnittansicht durch ein bevorzugtes Ausführungsbeispiel einer orthopädischen Fixationseinrichtung;
- Figur 3:: eine Draufsicht auf ein bevorzugtes Ausführungsbeispiel eines Querträgers mit einem durch einen Exzenter verschiebbaren Klemmstück in gelöster Stellung;
- Figur 4:: eine Ansicht ähnlich Figur 3 mit dem Klemmstück in gespannter Stellung;
- Figur 5:: eine Schnittansicht längs Linie 5-5 Figur 4;
- Figur 6:: eine Schnittansicht längs Linie 6-6 in Figur 4;
- Figur 7:: eine Ansicht ähnlich Figur 5 bei einem Fixationsglied mit einem Kugelkopf;
- Figur 8:: eine Ansicht ähnlich Figur 5 bei einem anderen bevorzugten Ausführungsbeispiel mit einem Exzenter in Form eines drehbaren Kernes und einem diesen umgebenden, mit dem Querträger fest verbundenen Lagerkörper;
- Figur 9:: eine weitere bevorzugte Ausführungsform mit einem Lagerkörper, der gleichzeitig den Exzenter zum Spannen des Klemmstückes trägt;
- Figur 10:: eine weitere bevorzugte Ausführungsform ähnlich dem der Figur 9 mit einer Kurvenbahn zur axialen Verschiebung des Klemmstückes und
- Figur 11:: eine weitere bevorzugte Ausführungsform einer orthopädischen Fixationseinrichtung mit einem Fixationsglied in Form eines Hakens.

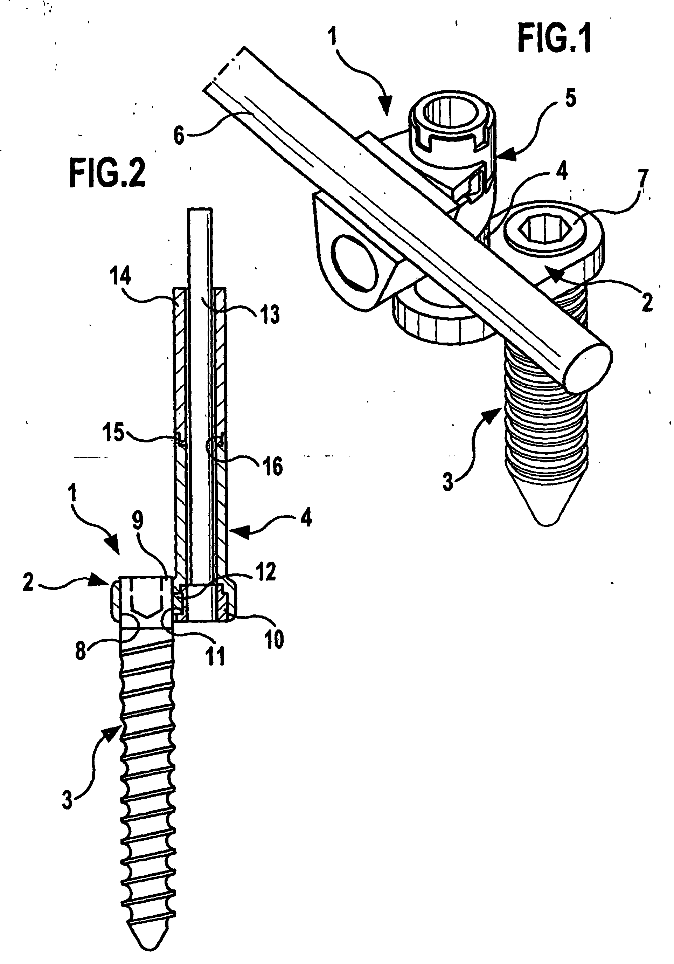

Die in Figur 1 dargestellte Fixationseinrichtung 1 umfaßt eine Knochenschraube 3, mit einem plattenförmigen, am oberen Ende der Knochenschraube 3 festlegbaren Querträger 2, der seinerseits einen hülsenförmigen, kreiszylindrischen Lagerkörper 4 trägt. Die Knochenschraube 3 und der Lagerkörper 4 sind parallel zueinander und durch den Querträger 2 seitlich versetzt zueinander angeordnet.

Auf den Lagerkörper kann eine in Figur 1 dargestellte und nicht näher erläuterte Haltevorrichtung 5 aufgesetzt sein, welche beispielsweise ein stabförmiges Verbindungselement 6 am Lagerkörper 4 festlegt, welches die dargestellte Fixationseinrichtung 1 mit einer ähnlich aufgebauten Fixationseinrichtung verbindet, wobei beide Fixationseinrichtungen über ihre Knochenschrauben 3 an unterschiedlichen Knochen oder Knochenfragmenten befestigt sind, die dadurch relativ zueinander positioniert und fixiert werden.

Der Querträger 2 ist an der Knochenschraube 3 um die Längsachse der Knochenschraube 3 verdrehbar gelagert und kann durch geeignete Mittel in unterschiedlichen Winkelstellungen relativ zur Knochenschraube 3 festgelegt werden. Beispielsweise kann dies dadurch erfolgen, daß ein an der Knochenschraube 3 festgelegter Klemmring 7 in Längsrichtung der Knochenschraube 3 gegen den Querträger 2 gepreßt wird, dieser kann beispielsweise selbst in die Knochenschraube 3 einschraubbar sein. Dies ist in der Darstellung der Figur 1 nur sehr schematisch gezeigt, man erkennt aber aus dieser Darstellung ohne weiteres, daß durch Verdrehung des Querträgers 2 relativ zur Knochenschraube 3 die gesamte Fixationseinrichtung 1 in ihrer Lage gegenüber der Knochenschraube 3 und damit gegenüber dem Knochenteil, in welches die Knochenschraube 3 eingeschraubt wird, in großem Umfange verstellt werden kann, ohne daß dabei die Knochenschraube 3 gegenüber dem Knochenteil in seiner Lage verändert werden muß.

Bei dem Ausführungsbeispiel der Figur 2 weist der Querträger eine Durchstecköffnung 8 auf, durch die ein zylindrischer Schaft 9 der Knochenschraube 3 hindurchgesteckt ist. Der Lagerkörper 4 ist ebenso wie beim Ausführungsbeispiel der Figur 1 als Hülse ausgebildet und fest mit dem Querträger 2 verbunden. Neben der Durchstecköffnung 8 ist konzentrisch zum Lagerkörper 4 ein Ring 10 im Querträger 2 verdrehbar gelagert, dessen Außenfläche 11 exzentrisch zu seiner Drehachse ausgebildet ist. Zwischen der Außenfläche 11 dieses Ringes 10 und der Durchstecköffnung 8 ist im Inneren des Querträgers 2 ein Klemmstück 12 verschieblich gelagert, welches beim Verdrehen des Ringes 10 durch die Exzentrizität der Außenfläche 11 gegen den Schaft 9 der in die Durchstecköffnung 8 eingeschobenen Knochenschraube 3 gedrückt werden kann, so daß dadurch dieser Schaft 9 in der Durchstecköffnung 8 festgeklemmt wird.

Zur Verdrehung des Ringes 10 kann in den hülsenförmigen Lagerkörper 4 ein Drehwerkzeug 13 eingeführt werden, welches drehfest in den Ring 10 eingreift. Außerdem ist ein Gegenhalterwerkzeug 14 vorgesehen, welches als Hülse ausgebildet ist, das Drehwerkzeug 13 umgibt und von oben her auf den ebenfalls hülsenförmigen Lagerkörper 4 aufgeschoben wird. Mit diesem geht es durch den Eingriff von Vorsprüngen 15 in Rücksprünge 16 eine drehfeste Verbindung ein, die es ermöglicht, beim Verdrehen des Drehwerkzeuges 13 den Lagerkörper 4 mittels des Gegenhalterwerkzeuges 14 festzuhalten, so daß keine unerwünschten Drehmomente auf den Querträger 2 übertragen werden.

Die in Figur 2 dargestellte Anordnung ist besonders kompakt aufgebaut, da die Längsachse des Lagerkörpers 4 mit der Drehachse des Ringes 10 zusammenfällt, man erkennt aus der Darstellung der Figur 2, daß der Querträger so kurz gebaut werden kann, daß Knochenschraube 3 und Lagerkörper 4 unmittelbar nebeneinander liegen können.

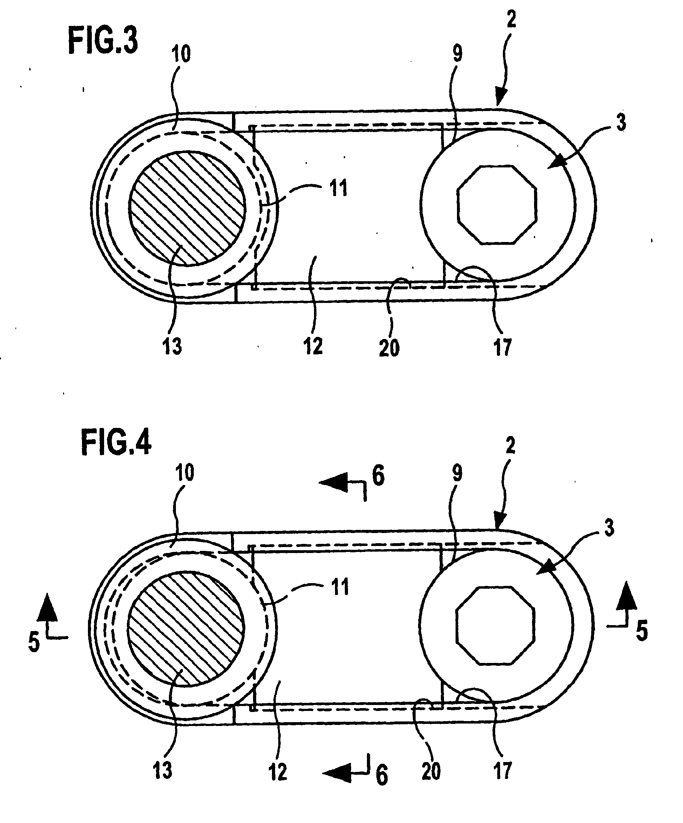

Bei anderen Ausführungsformen kann der Querträger 2 auch länger ausgebildet sein, dann wird auch das Klemmstück 12 entsprechend länger ausgebildet, wie dies am Beispiel der Figuren 3 bis 5 dargestellt ist. Dieser Querträger 2 ist als flache, streifenförmige Platte ausgebildet und trägt ein sich über einen großen Teil seiner Länge erstreckendes Langloch 17. Durch dieses Langloch 17 ist der Schaft 9 der Knochenschraube 3 hindurchgesteckt, auf der anderen Seite ist der Ring 10 in dem Langloch 17 drehbar gelagert und durch einen unteren Ringflansch 18 und einen oberen Ringflansch 19 in axialer Richtung gesichert. Zwischen dem Schaft 9 und dem Ring 10 ist auch hier ein im Langloch längsverschieblich geführtes Klemmstück 12 gehalten, welches sich einerseits an die exzentrische Außenfläche 11 des Ringes 10 und andererseits an den Schaft 9 anlegt.

Bei diesem Ausführungsbeispiel ist der hülsenförmige Lagerkörper 4 nicht dargestellt, es ist jedoch möglich, diesen den Ring 10 umgebend am Querträger 2 vorzusehen.

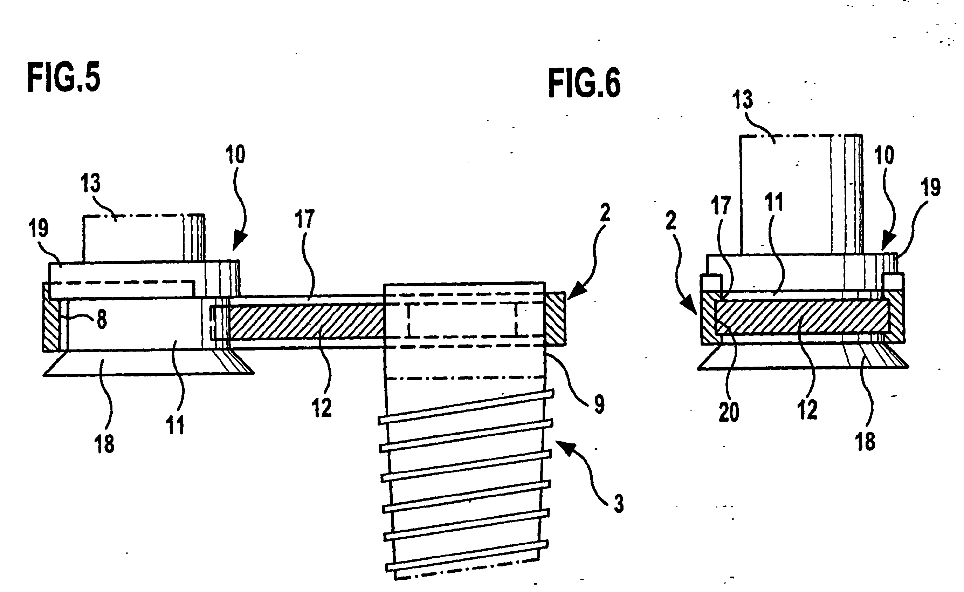

Aus der Darstellung der Figur 6 ist gut zu erkennen, daß das Klemmstück 12 in dem Langloch 17 in seitlichen Nuten 20 des Querträgers 2 in dessen Längsrichtung geführt ist, so daß das Klemmstück 12 im Querträger 2 unverlierbar angeordnet ist.

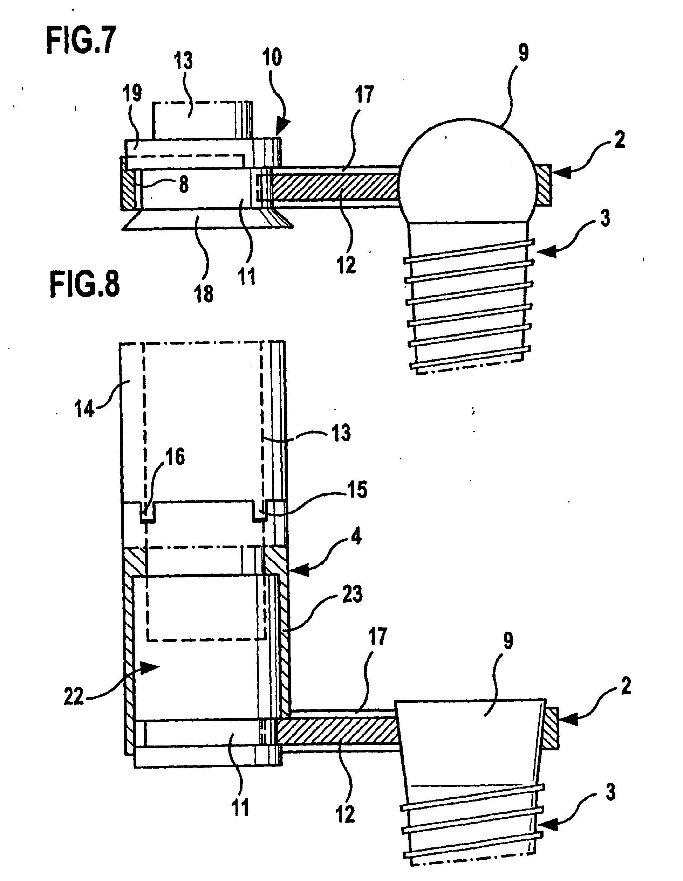

Im Ausführungsbeispiel der Figur 7 ist eine Knochenschraube 3 dargestellt, deren Schaft 9 nicht kreiszylindrisch ausgebildet ist, sondern kugelförmig. Dadurch besteht für den Querträger 2 ein zusätzlicher Freiheitsgrad, zwischen Querträger 2 und Knochenschraube 3 ist ein Kugelgelenk ausgebildet, das jedoch in gleicher Weise durch Verschiebung des Klemmstückes 12 gegen den Schaft 9 festgelegt, werden kann.

Beim Ausführungsbeispiel der Figur 8, das ähnlich aufgebaut ist wie das der Figuren 3 bis 7 und bei dem gleiche Teile dieselben Bezugszeichen tragen, ist eine Knochenschraube 3 mit einem sich nach oben hin konisch erweiternden Schaft 9 dargestellt, diese Schaftformen der Knochenschrauben 3 sind zwischen den verschiedenen Ausführungsbeispielen selbstverständlich austauschbar.

Außerdem ist bei dem Ausführungsbeispiel der Figur 8 gezeigt, daß die exzentrische-Außenfläche 11 hier nicht an einem Ring angeordnet ist, sondern an einem drehbaren Kern 22, der nach oben hin vom Querträger 2 absteht und in eine mit dem Querträger 2 fest verbundene, beispielsweise verschweißte Hülse 23 hineinragt, welche in diesem Falle den Lagerkörper 4 ausbildet. Zur Verdrehung kann wie beim Ausführungsbeispiel der Figur 2 von oben her ein Drehwerkzeug 13 in die Hülse 23 eingeschoben werden und dort durch einen geeigneten Formschluß eine drehfeste Verbindung zum Kern 22 herstellen.

Im Ausführungsbeispiel der Figur 8 ist auch ein Gegenhalterwerkzeug 14 dargestellt, welches ähnlich wie die Hülse 23 hülsenförmig ausgebildet ist und von oben her auf diese so aufgesetzt werden kann, daß Vorsprünge 15 am Gegenhalterwerkzeug 14 in entsprechende Rücksprünge 16 an der Hülse 23 eingreifen und dadurch eine drehfeste Verbindung herstellen. Das Gegenhalterwerkzeug 14 ermöglicht es, die Hülse 23 beim Verdrehen des Kernes 22 festzuhalten, so daß keine unerwünschten Drehmomente auf den Querträger 2 übertragen werden.

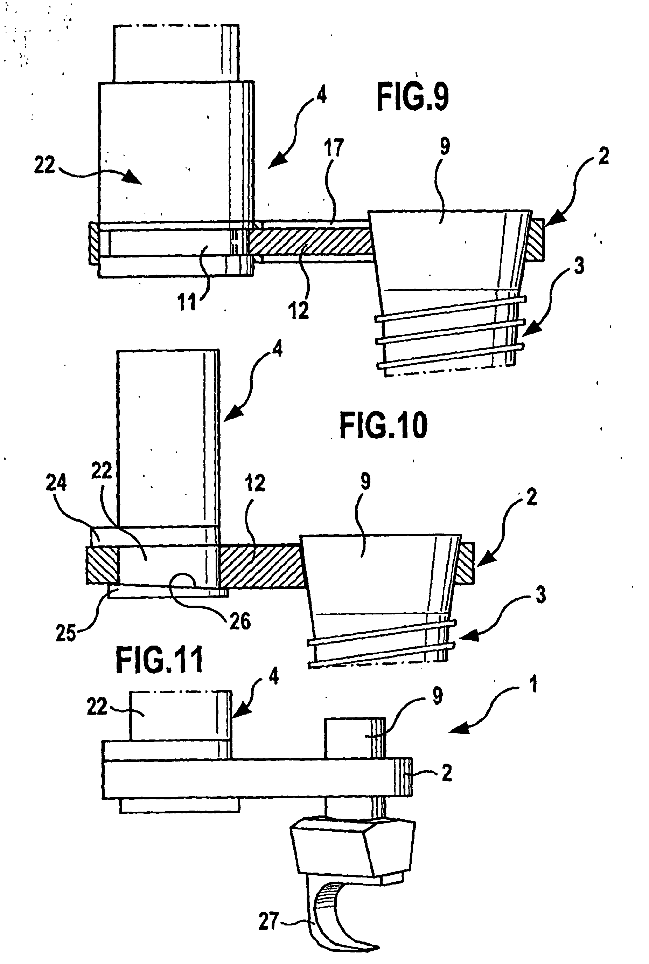

Bei dem Ausführungsbeispiel der Figur 9 fehlt ein Lagerkörper in Form einer Hülse, statt dessen bildet der Kern 22 selbst den Lagerkörper 4 aus. Im Ausführungsbeispiel der Figur 9 ist dieser Kern 22 stufig ausgebildet, es wäre aber auch möglich, diesen durchgehend kreiszylindrisch auszubilden. In diesem Fall dient der Kern 22 selbst zur Halterung für weitere orthopädische Fixationselemente.

Bei den Ausführungsbeispielen der Figuren 1 bis 9 wird das Klemmstück 12 durch einen Exzenter 11 gegen die Knochenschraube 3 gedrückt, die dadurch gegen eine Verdrehung und gegebenenfalls auch eine axiale Verschiebung gespannt wird.

Beim Ausführungsbeispiel der Figur 10 hingegen, bei dem wieder entsprechende Teile dieselben Bezugszeichen tragen, fehlt ein solcher Exzenter. Statt dessen ist der drehbar im Querträger 2 gelagerte Lagerkörper 4 durch zwei Ringflansche 24, 25 axial im Querträger 2 gesichert. Der Schaft 9 der Knochenschraube 3 erweitert sich nach oben hin konisch, und das dazwischenliegende Klemmstück 12 ist an diese Kontur angepaßt. Durch aus der Zeichnung nicht ersichtliche Mittel ist außerdem die Knochenschraube 3 in axialer Richtung im Querträger 2 gesichert, sie ist in diesem jedoch frei drehbar.

Der untere Ringflansch 25 untergreift das Klemmstück 12 mit seiner Oberseite, diese ist gegenüber einer Ebene geringfügig geneigt, die senkrecht auf der Drehachse des als Kern 22 ausgebildeten Lagerkörpers 4 steht. Damit bildet die Oberseite des unteren Ringflansches 25 eine geneigte Bahnkurve 26 aus, die bei Verdrehung des Lagerkörpers 4 das Klemmstück 12 parallel zur Drehachse verschiebt. Dabei verspannt es durch die konische Ausgestaltung des Schaftes 9 die Knochenschraube 3 und legt diese Knochenschraube 3 dadurch gegen Verdrehung fest. Während also bei dem Exzenter 11 die Verschiebung des Klemmstückes quer zur Drehachse des Exzenters erfolgt, wird bei dieser Ausgestaltung das Klemmstück parallel zur Drehachse des Lagerkörpers 4 verschoben. Diese Ausgestaltung kann bei allen Ausführungsformen, die vorstehend beschrieben sind, statt des Exzenters verwendet werden, es können auch beide Wirkungen kombiniert werden.

In Figur 11 ist ein weiteres abgewandeltes Ausführungsbeispiel einer Fixationseinrichtung 1 dargestellt, diese kann im wesentlichen gleich aufgebaut sein, wie die vorstehend beschriebenen Fixationseinrichtungen, gleiche Teile tragen daher dieselben Bezugszeichen. Im Unterschied zu den Ausführungsbeispielen der Figuren 1 bis 10 wird das Fixationsglied in diesem Fall nicht durch eine Knochenschraube gebildet, sondern durch einen Haken 27, der beispielsweise eine knöcherne Struktur im Körper eines Patienten umgreifen kann. Dieser Haken 27 trägt einen Schaft 9, der in gleicher Weise wie der Schaft 9 der Knochenschraube 3 der vorstehend beschriebenen Ausführungsbeispiele gegenüber einem Querträger 2 frei verdrehbar und sowohl hinsichtlich der Winkelstellung als auch gegebenenfalls hinsichtlich der Axialposition festklemmbar ist.

## Patentansprüche

1. Orthopädische Fixationseinrichtung (1) mit einem zumindest abschnittsweise schaftförmigen Fixationsglied (3, 27), einem schaftförmigen Lagerkörper (4) und mit einem das Fixationsglied (3, 27) und den Lagerkörper (4) seitlich versetzt zueinander festlegenden Querträger (2), in dem das Fixationsglied (3, 27) um seine Längsachse verdrehbar und in einer bestimmten Winkelstellung festklemmbar ist, **dadurch gekennzeichnet, daß** zur Festklemmung im Querträger (2) ein Klemmstück (12) gegen das Fixationsglied (3, 27) verschiebbar ist.

2. Einrichtung nach Anspruch 1, **dadurch gekennzeichnet, daß** zur Verschiebung des Klemmstückes (12) im Querträger (2) ein am Klemmstück (12) anliegendes, um eine Drehachse verdrehbares Spannglied (11; 26) gelagert ist.

3. Einrichtung nach Anspruch 2, **dadurch gekennzeichnet, daß** die Drehachse des Spanngliedes (11; 26) senkrecht auf der Ebene des Querträgers (2) steht.

4. Einrichtung nach Anspruch 3, **dadurch gekennzeichnet, daß** die Drehachse des Spanngliedes (11; 26) parallel zur Längsachse des Schaftabschnittes des Fixationsgliedes (3, 27) verläuft.

5. Einrichtung nach einem der Ansprüche 1 bis 4, **dadurch gekennzeichnet, daß** der Querträger (2) ein Langloch (17) aufweist, in welchem das Fixationsglied (3, 27), das Klemmstück (12) und das Spannglied (11; 26) aufgenommen sind.

6. Einrichtung nach einem der Ansprüche 2 bis 5, **dadurch gekennzeichnet, daß** die Drehachse des Spanngliedes (11; 26) und die Längsachse des Lagerkörpers (4) zusammenfallen.

7. Einrichtung nach Anspruch 6, **dadurch gekennzeichnet, daß** der Lagerkörper (4) eine fest mit dem Querträger (2) verbundene Hülse (23) ist, durch die hindurch ein Werkzeug (13) zur Verdrehung des Spanngliedes (11; 26) einsetzbar ist.

8. Einrichtung nach Anspruch 7, **dadurch gekennzeichnet, daß** der Lagerkörper (4) Aufnahmen (16) für ein Gegenhalterwerkzeug (14) aufweist.

9. Einrichtung nach Anspruch 7 oder 8, **dadurch gekennzeichnet, daß** das Spannglied (11; 26) an einem im Querträger (2) verdrehbaren Ring (10) angeordnet ist, der sich am querträgerseitigen Ende des Lagerkörpers (4) befindet.

10. Einrichtung nach Anspruch 7 oder 8, **dadurch gekennzeichnet, daß** das Spannglied (11; 26) an einem im Lagerkörper (4) drehbar gelagerten Kern (22) angeordnet ist, der sich zumindest über einen Teil seiner Länge innerhalb des Lagerkörpers (4) erstreckt.

11. Einrichtung nach Anspruch 7, **dadurch gekennzeichnet, daß** der Lagerkörper (4) im Querträger (2) um seine Längsachse verdrehbar ist und selbst das Spannglied (11; 26) trägt.

12. Einrichtung nach einem der Ansprüche 2 bis 11, **dadurch gekennzeichnet, daß** das Spannglied (11; 26) im Querträger (2) in axialer Richtung festgelegt ist.

13. Einrichtung nach einem der Ansprüche 2 bis 12, **dadurch gekennzeichnet, daß** das Spannglied einen zu seiner Drehachse exzentrisch angeordneten Exzenter (11) aufweist, der an dem Klemmstück (12) anliegt.

14. Einrichtung nach einem der Ansprüche 2 bis 12, **dadurch gekennzeichnet, daß** das Spannglied eine geneigte, am Klemmstück (12) anliegende Bahnkurve (26) aufweist.

15. Einrichtung nach Anspruch 14, **dadurch gekennzeichnet, daß** die Bahnkurve (26) das Klemmstück (12) bei Verdrehung des axial unverschieblichen Spanngliedes parallel zur Drehachse gegen das Fixationsglied (3, 27) verschiebt und dieses **dadurch** festklemmt.

16. Einrichtung nach Anspruch 15, **dadurch gekennzeichnet, daß** das Fixationsglied (3, 27) einen sich erweiternden Schaft (9) aufweist, an dem das Klemmstück (12) anliegt, und axial unverschieblich und frei drehbar im Querträger (2) gelagert ist.

17. Einrichtung nach einem der voranstehenden Ansprüche, **dadurch gekennzeichnet, daß** das Fixationsglied (3, 27) durch eine kugelige Verbindung mit dem Querträger (2) verschwenkbar verbunden ist und das Klemmstück (12) beim Fixieren in Richtung auf eine kugelige Lagerfläche (9) des Fixationsgliedes (3, 27) verschiebbar ist.

18. Einrichtung nach einem der voranstehenden Ansprüche, **dadurch gekennzeichnet, daß** das Fixationsglied eine Knochenschraube (3) ist.

19. Einrichtung nach einem der Ansprüche 1 bis 17, **dadurch gekennzeichnet, daß** das Fixationsglied ein Haken (27) ist.

## Claims

1. Orthopedic fixation device (1) with an at least partly shaft-like fixation member (3, 27), with a shaft-like mounting body (4), and with a transverse support (2) securing the fixation member (3, 27) and the mounting body (4) in a position in which they are laterally offset from one another, in which transverse support (2) the fixation member (3, 27) can be rotated about its longitudinal axis and can be clamped in a defined angular position, **characterized in that**, in order to permit clamping in the transverse support (2), a clamp piece (12) can be moved against the fixation member (3, 27).

2. Device according to Claim 1, **characterized in that**, in order to move the clamp piece (12) in the transverse support (2), a tensioning member (11; 26) is mounted so that it engages against the clamp piece (12) and is rotatable about a rotation axis.

3. Device according to Claim 2, **characterized in that** the rotation axis of the tensioning member (11; 26) is perpendicular to the plane of the transverse support (2).

4. Device according to Claim 3, **characterized in that** the rotation axis of the tensioning member (11; 26) extends parallel to the longitudinal axis of the shaft portion of the fixation member (3, 27).

5. Device according to one of Claims 1 to 4, **characterized in that** the transverse support (2) has an oblong hole (17) in which the fixation member (3, 27), the clamp piece (12) and the tensioning member (11; 26) are accommodated.

6. Device according to one of Claims 2 to 5, **characterized in that** the rotation axis of the tensioning member (11; 26) and the longitudinal axis of the mounting body (4) coincide.

7. Device according to Claim 6, **characterized in that** the mounting body (4) is a sleeve (23) which is fixedly connected to the transverse support (2) and through which a tool (13) can be inserted for rotating the tensioning member (11; 26).

8. Device according to Claim 7, **characterized in that** the mounting body (4) has seats (16) for a stabilizing tool (14).

9. Device according to Claim 7 or 8, **characterized in that** the tensioning member (11; 26) is disposed on a ring (10) which is rotatable in the transverse support (2) and which is located at that end of the mounting body (4) on the transverse support.

10. Device according to Claim 7 or 8, **characterized in that** the tensioning member (11; 26) is disposed on a core (22) which is mounted rotatably in the mounting body (4) and which, at least along part of its length, extends inside the mounting body (4).

11. Device according to Claim 7, **characterized in that** the mounting body (4) can rotate about its longitudinal axis in the transverse support (2) and itself carries the tensioning member (11; 26).

12. Device according to one of Claims 2 to 11, **characterized in that** the tensioning member (11; 26) is secured in the axial direction in the transverse support (2).

13. Device according to one of Claims 2 to 12, **characterized in that** the tensioning member has a cam (11) which is disposed eccentrically with respect to its rotation axis and engages against the clamp piece (12).

14. Device according to one of Claims 2 to 12, **characterized in that** the tensioning member has an inclined path curve (26) engaging against the clamp piece (12).

15. Device according to Claim 14, **characterized in that**, when the axially immovable tensioning member is rotated parallel to the rotation axis, the cam (26) pushes the clamp piece (12) against the fixation member (3, 27) and thereby clamps the latter.

16. Device according to Claim 15, **characterized in that** the fixation member (3, 27) has a widening shaft (9), against which the clamp piece (12) engages, and is mounted so as to be axially immovable and freely rotatable in the transverse support (2).

17. Device according to one of the preceding claims, **characterized in that** the fixation member (3, 27) is connected pivotably to the transverse support (2) via a spherical connection, and the clamp piece (12) can, at the time of fixing, be moved in the direction of a spherical bearing surface (9) of the fixation member (3, 27).

18. Device according to one of the preceding claims, **characterized in that** the fixation member is a bone screw (3).

19. Device according to one of Claims 1 to 17, **characterized in that** the fixation member is a hook (27).

## Revendications

1. Système de fixation orthopédique (1) comportant un organe de fixation (3, 27) en forme de tige au moins par tronçons, un corps de montage (4) en forme de tige et comportant un support transversal (2) immobilisant l'un avec l'autre en décalage latéral l'organe de fixation (3, 27) et le corps de montage (4), support transversal dans lequel l'organe de fixation (3, 27) peut tourner autour de son axe longitudinal et peut être bloqué dans une position angulaire déterminée, **caractérisé en ce que** pour effectuer le blocage, une pièce de blocage (12) dans le support transversal (2) peut être déplacé en translation contre l'organe de fixation (3, 27).

2. Système selon la revendication 1, **caractérisé en ce que** pour déplacer la pièce de blocage (12) dans le support transversal (2) est prévu un organe de serrage (11 ; 26) monté en appui sur la pièce de blocage (12) et susceptible de tourner autour d'un axe de rotation.

3. Système selon la revendication 2, **caractérisé en ce que** l'axe de rotation de l'organe de serrage (11 ; 26) est perpendiculaire au plan du support transversal (2).

4. Système selon la revendication 3, **caractérisé en ce que** l'axe de rotation de l'organe de serrage (11 ; 26) s'étend parallèlement à l'axe longitudinal du tronçon de tige de l'organe de fixation (3, 27).

5. Système selon l'une des revendications 1 à 4, **caractérisé en ce que** le support transversal (2) présente un trou oblong (17) dans lequel sont reçus l'organe de fixation (3, 27), la pièce de blocage (12) et l'organe de serrage (11 ; 26).

6. Système selon l'une des revendications 2 à 5, **caractérisé en ce que** l'axe de rotation de l'organe de serrage (11 ; 26) et l'axe longitudinal du corps de montage (4) coïncident.

7. Système selon la revendication 6, **caractérisé en ce que** le corps de montage (4) est une douille (23) reliée de manière solidaire au support transversal (2), à travers laquelle on peut insérer un outil (13) pour faire tourner l'organe de serrage (11 ; 26).

8. Système selon la revendication 7, **caractérisé en ce que** le corps de montage (4) présente un logement (16) pour un outil de retenue antagoniste (14).

9. Système selon la revendication 7 ou 8, **caractérisé en ce que** l'organe de serrage (11 ; 26) est agencé sur une bague (10) susceptible de tourner dans le support transversal (2), laquelle se trouve à l'extrémité coté support transversal du corps de montage (4).

10. Système selon la revendication 7 ou 8, **caractérisé en ce que** l'organe de serrage (11 ; 26) est agencé sur un noyau (22) monté rotatif dans le corps de montage (4), lequel s'étend au moins sur une partie de sa longueur à l'intérieur du corps de montage (4).

11. Système selon la revendication 7, **caractérisé en ce que** le corps de montage (4) est susceptible de tourner autour de son axe longitudinal dans le support transversal (2) et porte lui-même l'organe de serrage (11 ; 26).

12. Système selon l'une des revendications 2 à 11, **caractérisé en ce que** l'organe de serrage (11 ; 26) est immobilisé en direction axiale dans le support transversal (2).

13. Système selon l'une des revendications 2 à 12, **caractérisé en ce que** l'organe de serrage présente un excentrique (11) agencé excentriquement par rapport à son axe de rotation, lequel est en appui sur la pièce de blocage (12).

14. Système selon l'une des revendications 2 à 12, **caractérisé en ce que** l'organe de serrage présente une came (26) inclinée en appui sur la pièce de blocage (12).

15. Système selon la revendication 14, **caractérisé en ce que** lorsque l'organe de serrage axialement indéplaçable tourne, la came (26) déplace la pièce de blocage (12) parallèlement à l'axe de rotation contre l'organe de fixation (3, 27) et bloque ainsi celui-ci.

16. Système selon la revendication 15, **caractérisé en ce que** l'organe de fixation (3, 27) présente une tige (9) qui s'élargit, sur laquelle est en appui la pièce de blocage (12) et qui est montée de manière axialement indéplaçable et librement rotative dans le support transversal (2).

17. Système selon l'une des revendications précédentes, **caractérisé en ce que** l'organe de fixation (3, 27) est relié à pivotement avec le support transversal (2) par une liaison sphérique et lors de la fixation, la pièce de blocage (12) est déplaçable en direction d'une surface d'appui (9) sphérique de l'organe de fixation (3, 27).

18. Système selon l'une des revendications précédentes, **caractérisé en ce que** l'organe de fixation est une vis à os (3).

19. Système selon l'une des revendications 1 à 17, **caractérisé en ce que** l'organe de fixation est un crochet (27).
